# FASCICULE DE BREVET EUROPEEN

(11) **EP 2 861 275 B1**
(45) Date de publication et mention de la délivrance du brevet: **31.10.2018**
(21) Numéro de dépôt: 13728202.6
(22) Date de dépôt: 13.06.2013
(51) Int. Cl.: A61M 39/00, A61M 39/24, A61M 5/00, A61M 5/148

(54) **DISPOSITIF D'INJECTION D'UN PRODUIT LIQUIDE COMPRENANT DEUX DEMI-COQUES MOBILES EN ROTATION L'UNE PAR RAPPORT A L'AUTRE**
VORRICHTUNG ZUR INJEKTION EINES FLÜSSIGPRODUKTS MIT ZWEI GEGENEINANDER DREHBAREN HALBSCHALEN
DEVICE FOR INJECTING A LIQUID PRODUCT COMPRISING TWO HALF-SHELLS ROTATABLY MOBILE RELATIVE TO EACH OTHER

(30) Priorité: 13.06.2012 FR 1255530; 13.06.2012 US 201261659288 P
(43) Date de publication de la demande: 22.04.2015
(73) Titulaire: Medex, 69800 Saint-Priest (FR)
(72) Inventeur: REBERGUE, Habib, 69008 Lyon (FR); TERRASSE, Samuel, 38080 Saint Alban De Roche (FR); MATRAY, Damien, 38300 Bourgoin-Jallieu (FR)
(74) Mandataire: Regimbeau
(86) Numéro de dépôt international: PCT/EP2013/062214
(87) Numéro de publication internationale: WO 2013/186289

(56) Documents cités:
- EP-A1- 0 676 214
- WO-A1-93/25269
- WO-A1-03/039433
- WO-A1-2005/072666
- FR-A1- 2 154 675
- US-A- 5 348 539
- US-A- 5 368 569
- US-A1- 2008 275 590
- US-A1- 2011 196 304

## Description

### DOMAINE TECHNIQUE

La présente invention concerne le domaine technique général des dispositifs d'injection de produit de contraste.

### PRESENTATION GENERALE DE L'ART ANTERIEUR

Les développements de la médecine ont conduit à mettre au point différents procédés d'analyse et de contrôle de l'état des patients. Parmi ces procédés, il existe les analyses faites après injection d'un produit de contraste, par exemple pour l'imagerie médicale qui regroupe aussi bien l'imagerie par rayons X que l'imagerie par Résonance Magnétique (IRM) ou la médecine nucléaire.

On connaît des dispositifs pour injecter un produit liquide tel qu'un produit de contraste à un patient comme par exemple les techniques de pompe en ligne, les techniques dites « *pousse-seringue »,* ou encore les techniques d'injecteur à poche.

Le document EP 0 676 214 décrit un dispositif d'injection comportant deux coques mobiles en rotation l'une par rapport à l'autre. Chaque coque comprend une cavité recouverte d'une membrane souple. Chaque coque est alimentée en fluide hydraulique moteur par un vérin hydraulique connecté auxdites coques par l'intermédiaire de flexibles d'alimentation hydraulique. D'autres documents décrivent un dispositif d'injection d'un produit liquide contenu dans une poche et les dispositifs comprenant au moins une vessie active déformable à volume variable correspondent aux documents suivants: FR 2 154 675, US 2011/196304, US 2008/275590, WO 03/039433, US 5 348 539, WO 2005/072666, US 5 368 569 et WO 93/25269.

Un but de la présente invention est de proposer un dispositif d'injection amélioré comportant deux demi-coques mobiles en rotation l'une par rapport à l'autre.

### PRESENTATION DE L'INVENTION

A cet effet, l'invention propose un dispositif pour l'injection d'un produit liquide contenu dans une poche, le dispositif comprenant un boîtier constitué de deux demi-coques articulées afin de permettre le déplacement relatif des demi-coques l'une par rapport à l'autre entre :
- une position ouverte pour la mise en place de la poche et
- une position fermée pour l'injection du produit liquide contenu dans la poche,
remarquable en ce que :
- l'une des demi-coques comprend une vessie - dite « vessie active » - déformable à volume variable sous l'action d'une source de puissance hydraulique alimentant ladite vessie en fluide hydraulique, et
- l'autre des demi-coques comprend un coussin déformable à volume constant - dit « coussin passif ».

Le fait que le dispositif comprenne une vessie active d'une part et un coussin passif d'autre part permet de diminuer l'encombrement du dispositif d'injection puisqu'il n'est pas nécessaire de prévoir de flexibles d'alimentation hydraulique au niveau des deux demi-coques. Ceci permet également de faciliter la manipulation du dispositif d'injection puisqu'il n'est plus nécessaire pour l'utilisateur d'assister le déplacement des flexibles d'alimentation hydraulique des deux demi-coques lors du déplacement relatifs de celles-ci l'une par rapport à l'autre.

Le fait que l'une des demi-coques comprenne un coussin passif déformable à volume constant présente de nombreux avantages par rapport à un dispositif dépourvu de coussin passif :
- Le fait que le coussin passif soit déformable permet de garantir un contact optimal du coussin sur la poche de produit liquide. Par « contact optimal », on entend une coïncidence de plus de 80%, voire plus de 90% entre la surface du coussin passif et la surface de la poche pouvant entrer en contact l'une avec l'autre. Le caractère optimal de ce contact permet d'assurer un contrôle des paramètres d'injection de produit liquide. De préférence, le coussin passif est fortement déformable (i.e. qu'il présente une dureté inférieure à 10 Shore A (la dureté Shore, exprimée en Shore A ou ShA, correspond à une unité de mesure de la dureté d'élastomères ou de certaines matières plastiques très bien connue de l'homme du métier et reconnue par les normes internationales ISO 868 et 7619, ASTM D 2240 et DIN 53505). Ce caractère « fortement déformable » du coussin permet d'induire une déformation du coussin passif pour une faible pression dans le récipient. On limite ainsi :
   ∘ la déformation nécessaire de la vessie active pour induire le placage de la poche contre le coussin passif, et
   ∘ les efforts subis par la poche de produit liquide.
   ∘ et ainsi, indirectement le risque de rupture de la membrane de la vessie active et/ou de la poche.
- Le fait que la déformation du coussin passif soit à volume constant permet l'estimation de la déformation du système. En effet, dans le cas d'un coussin passif à volume variable (par exemple incluant un gel compressible), il serait nécessaire d'augmenter la pression à l'intérieur de la poche pour garantir un même niveau de contact du coussin passif sur la poche de produit liquide. Une telle utilisation d'un coussin passif à volume variable ne permet pas au dispositif d'injection de fonctionner selon une loi de déformation sous l'effet de la pression à l'intérieur de la poche. La répétabilité des conditions de l'injection serait alors difficile à assurer. D'autre part, l'utilisation d'un coussin passif à volume variable peut fausser les résultats d'une détection d'air dans l'unité de mise en pression du dispositif d'injection ou même empêcher la mise en oeuvre d'une telle détection.

Dans le cas d'un dispositif d'injection dépourvu de coussin passif déformable à volume constant (par exemple, incluant une contre-forme rigide), les problèmes suivants sont rencontrés :
- Usure prématurée de la vessie active,
- Impossibilité d'utiliser le dispositif d'injection pour différentes tailles de poche de produit liquide,
- Risque d'éclatement de la poche de produit liquide lors de sa mise en pression,
- Nécessité d'utiliser une valve à seuil d'ouverture élevé (c'est-à-dire supérieur à 500 mbar) entre la poche et le patient.

Ces problèmes sont liés aux difficultés rencontrées pour assurer un placage de la poche contre les parois du dispositif d'injection. Or, la qualité de ce placage de la poche contre les parois du dispositif d'injection est très importante pour contrôler précisément les paramètres (quantité injectée, débit, etc.) de l'injection de produit liquide.

Ainsi, dans le cas d'un dispositif d'injection incluant par exemple une vessie active sur l'une des demi-coques et une plaque rigide sur l'autre demi-coque, il est nécessaire que la vessie active applique une force importante sur la poche pour plaquer celle-ci sur toute sa surface contre la plaque rigide. Ceci induit la nécessité d'atteindre une pression supérieure dans la poche pour obtenir le même niveau de performance qu'avec le dispositif d'injection selon l'invention incluant un coussin passif déformable à volume constant. Le risque de rupture de la poche est alors accru dans ce type de dispositif.

Des aspects préférés mais non limitatifs du dispositif selon l'invention sont les suivants :
- chaque demi-coque comporte une cavité de sorte à former un berceau pour la vessie active ou le coussin passif. Ce berceau peut former un élément anti-extrusion pour la vessie active ou le coussin passif, par exemple si sa profondeur est plus grande que l'épaisseur dudit coussin passif. En effet, lors de la montée en pression à l'intérieur du dispositif d'injection, la déformation du coussin passif, en particulier, peut créer un jeu entre les pièces du dispositif (notamment les deux demi-coques) : ce jeu peut être à l'origine d'un risque d'extrusion, en particulier du coussin passif, qui est réduit par la présence d'un berceau tel que décrit ci-dessus,
   Dans un mode de réalisation préféré, on a ainsi une demi-coque comportant une cavité dans laquelle se trouve le coussin passif, celui-ci ayant une épaisseur inférieure à la profondeur de ladite cavité. On forme ainsi une bague « anti-extrusion » sur toute la périphérie de la jointure des deux demi-coques.
- la demi-coque comprenant la vessie active est fixe et la demi-coque comprenant le coussin passif est mobile en rotation,
- la vessie active comprend deux membranes superposées fixées à leurs périphéries de manière à obtenir un volume étanche destiné à recevoir le fluide hydraulique,
- la membrane destinée à venir en contact avec la demi-coque présente une rigidité supérieure à l'autre membrane destinée à venir en contact avec la poche,
- la dureté shore de la membrane destinée à venir en contact avec la demi-coque est comprise entre 70 et 90 shore A, la dureté shore de l'autre membrane étant comprise entre 20 et 50 shore A,
- la vessie comprend en outre un corps annulaire de renfort rigide s'étendant à la périphérie des membranes de la vessie,
- le coussin passif comprend une couche épaisse déformable (241) de dureté shore inférieure à 10 shore A,
- le coussin passif comprend en outre une couche rigide (242) superposée à la couche épaisse, la couche rigide étant destinée à venir en contact avec la demi-coque,
- le coussin passif comprend un élément chauffant positionné entre la couche rigide et la couche épaisse déformable,
- le coussin passif comprend en outre une structure de maintien rigide s'étendant à la périphérie de la couche épaisse déformable, notamment sur toute l'épaisseur de la couche épaisse et formant un élément anti-extrusion, Cette structure de maintien rigide joue donc un rôle de bague « anti-extrusion »,
- l'une des demi-coques comprend un logement comportant une paroi latérale, le logement étant destiné à recevoir un raccord de branchement d'un membre d'accès de la poche, la forme de la paroi latérale dudit logement présentant une symétrie de révolution,
- le logement comprend un guide, tel qu'une fente longitudinale, pour le passage du membre d'accès de la poche, le guide et la forme de la paroi latérale étant adaptés pour permettre le positionnement du raccord de branchement au niveau d'une base du logement par gravité,
- le logement comprend en outre une butée adaptée pour empêcher un mouvement du raccord de branchement selon une direction parallèle à un axe longitudinal du logement lorsque le raccord de branchement est en position dans le logement,
- le dispositif comprend en outre une unité de contrôle programmée pour :
   o recevoir une information sur la nature du produit liquide contenu dans la poche,
   ∘ déterminer un débit de sortie de référence en fonction de ladite information sur la nature du produit contenu dans la poche,
   ∘ estimer le débit de sortie du dispositif d'injection,
   ∘ comparer le débit de sortie de référence au débit de sortie estimé,
   ∘ émettre une alarme si la différence entre le débit de sortie estimé et le débit de sortie de référence est supérieure à une valeur seuil.

### PRESENTATION DES FIGURES

D'autres caractéristiques et avantages de l'invention ressortiront encore de la description qui suit, laquelle est purement illustrative et non limitative et doit être lue en regard des dessins annexés, sur lesquels :
- la figure 1 illustre une variante de réalisation d'un dispositif d'injection,
- les figures 2 et 3 illustrent deux exemples de poche contenant un produit liquide injectable,
- les figures 4 et 5 illustrent les positions fermée et ouverte d'un mode de réalisation d'une unité de mise en pression,
- la figure 6 illustre un exemple de demi-coque du dispositif illustré à la figure 1,
- les figures 7 et 8 illustrent une vessie du dispositif illustré à la figure 1,
- les figures 9 et 10 illustrent un coussin du dispositif illustré à la figure 1,
- la figure 11 illustre une vue en coupe de la vessie représentée aux figures 7 et 8,
- la figure 12 illustre un ensemble de connexion pour l'intégration d'un dispositif de remplissage à un injecteur à poche
- la figure 13 illustre un logement d'embout de poche.

### DESCRIPTION DE MODES DE REALISATION DE L'INVENTION

On va maintenant décrire plus en détail l'invention en référence aux figures. Dans ces différentes figures, les éléments équivalents portent les mêmes références numériques.

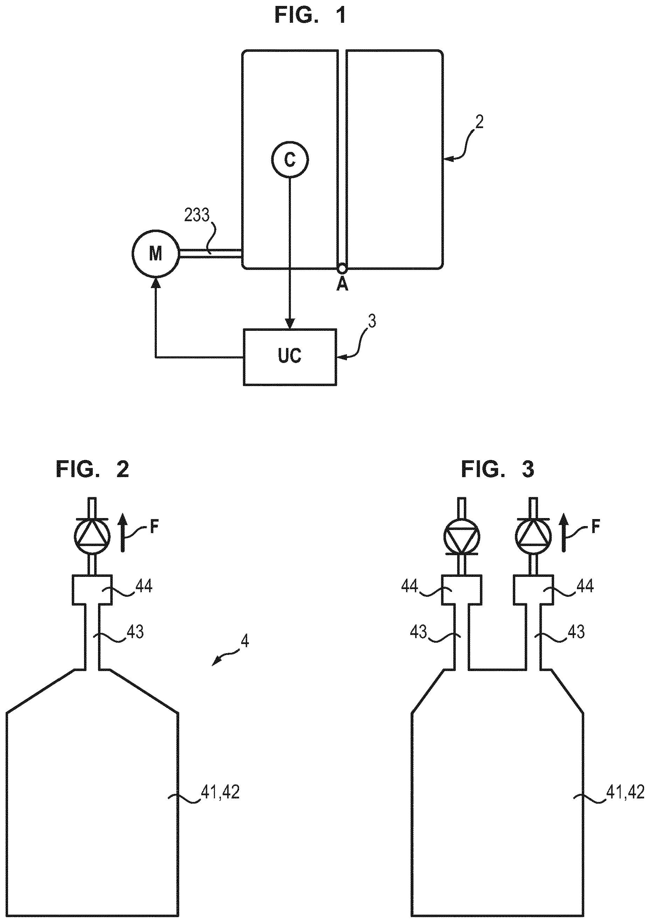

En référence à la figure 1, on a illustré un exemple de dispositif d'injection de type injecteur à poche. Le dispositif comprend une unité de mise en pression 2, une unité de contrôle 3.

Le dispositif d'injection permet l'injection d'un produit liquide contenu dans une poche 4 (cf. figures 2 et 3).

### Poche

En référence aux figures 2 et 3, la poche médicale 4 comporte deux feuilles superposées 41, 42 de longueur et de largeur appropriées ainsi qu'un (ou plusieurs) membre(s) d'accès 43.

Les feuilles 41, 42 sont faites de plusieurs couches de films minces stratifiées en matériaux flexibles ou souples, éventuellement transparents ou translucides tels que les matériaux polymères comprenant le polyéthylène, le polypropylène, et des matériaux de préférence thermoplastiques.

Les feuilles superposées 41, 42 sont de préférence soudées plates entre elles afin de former une poche 4. Les feuilles superposées 41, 42 sont scellées à leurs périphéries latérales pour former une poche 4 d'aspect général extérieur rectangulaire. Quand la poche médicale 4 est remplie ou partiellement remplie, elle présente la forme d'un coussin.

Un membre d'accès 43 est prévu au niveau de la partie supérieure de la poche 4. Le membre d'accès 43 est scellé entre les feuilles superposées 41, 42. Ce membre d'accès 43 est un tube et peut comprendre à son extrémité distale un raccord de branchement 44 pour le couplage de la poche à une tubulure reliée au patient.

Un autre membre d'accès 43 peut être prévu sur la poche. Dans ce cas :
- le premier membre d'accès - dit membre d'accès amont - est destiné à être connecté à une source contenant le produit liquide à injecter au patient pour permettre le remplissage de la poche,
- le deuxième membre d'accès - dit membre d'accès aval - est destiné à être connecté à une tubulure reliée au patient (par l'intermédiaire de plusieurs éléments tels qu'une canalisation et un cathéter ou une aiguille hypodermique/intraveineuse) pour l'injection au patient du produit liquide.

Avantageusement, une valve anti-retour à seuil peut être positionnée entre le membre d'accès aval et la tubulure reliée au patient. La valve anti-retour à seuil est adaptée pour autoriser le passage du liquide d'amont en aval comme représenté par la flèche "F" lorsqu'une pression déterminée du fluide en écoulement est atteinte, tandis qu'elle condamne le passage du liquide médical en sens inverse, à savoir, d'aval en amont, c'est-à-dire dans le sens contraire de celui illustré par la flèche "F".

Une autre valve anti-retour peut être positionnée entre le membre d'accès amont et la source pour autoriser le passage de produit liquide uniquement de la source vers la poche. On peut par exemple prévoir un système de valves tel que décrit dans le document EP 0 648 513.

Lorsque la poche comprend un unique membre d'accès 43, alors le membre d'accès joue les deux rôles cités précédemment, à savoir le remplissage et l'injection.

### Demi-coques

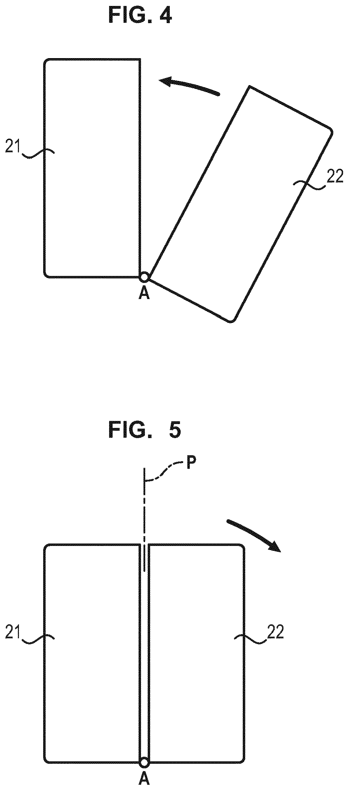

L'unité de mise en pression 2 comprend une enceinte rigide composée de deux demi-coques 21, 22 articulées autour d'un axe de rotation A-A' de sorte à permettre le déplacement relatif des demi-coques l'une par rapport à l'autre. Ces deux demi-coques 21, 22 sont aptes à être déplacées relativement l'une par rapport à l'autre entre :
- une position ouverte (figure 4) pour la mise en place de la poche et
- une position fermée (figure 5) pour l'injection du produit liquide contenu dans la poche.

De préférence l'une des demi-coques 21 est fixe et l'autre 22 est mobile en rotation selon l'axe A-A'.

Avantageusement, l'axe de rotation A-A' peut être décalé par rapport au centre de gravité G de la demi-coque mobile. Ceci permet d'obtenir une ouverture automatique de la demi-coque mobile 22 par gravité afin de limiter le nombre de manipulations nécessaires par l'utilisateur. De préférence, l'axe de rotation A-A' est le plus éloigné possible de la poche pour limiter les risques de frottements durant l'ouverture du dispositif.

De préférence, les demi-coques 21, 22 ne sont pas motorisées afin d'éviter les risques de pincement de l'utilisateur. Des ressorts (non représentés) peuvent être prévus entre les deux demi-coques pour assister l'utilisateur dans la fermeture de la porte mobile par compensation du poids de la demi-coque mobile. Ces ressorts peuvent jouer un rôle d'amortisseur pour ralentir la chute de la demi-coque mobile 22, chute qui pourrait être dangereuse pour l'utilisateur.

Chaque demi-coque comporte une cavité de sorte à former un berceau.

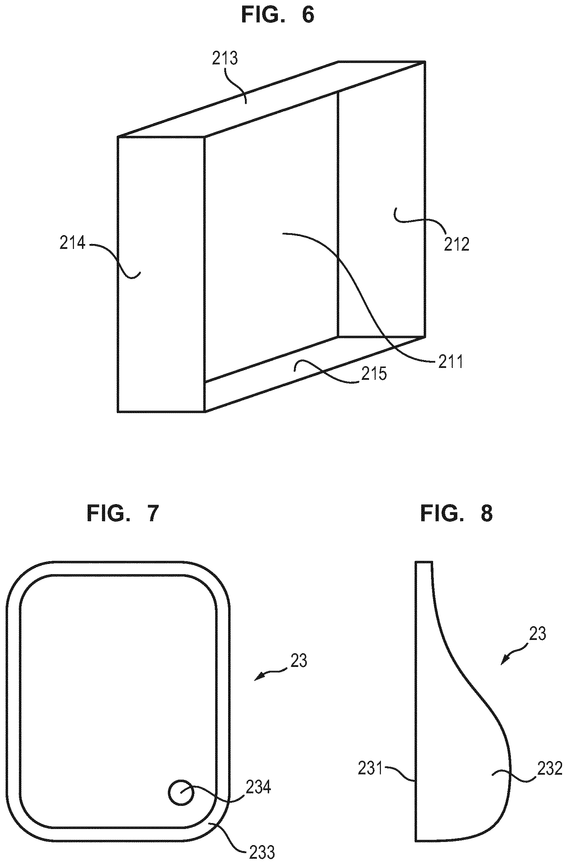

Dans le mode de réalisation illustré à la figure 6, chaque demi-coque 21, 22 comporte, une face arrière plane 211, une face avant et quatre faces latérales 212 à 215 s'étendant à la périphérie de la face arrière 211, perpendiculairement à celle-ci. La face avant est destinée à venir en contact avec une poche. La face avant peut présenter une forme concave de sorte à définir la cavité formant le berceau. En variante, une ou chaque demi-coque peut comporter une unique paroi concave définissant la cavité formant le berceau.

La cavité de l'une 21 des demi-coques - par exemple la demi-coque fixe - est destinée à recevoir une vessie 23 illustrée aux figures 7 et 8. La vessie 23 est composée d'au moins deux membranes 231, 232 soudées à leur périphérie. Ces membranes 231, 232 soudées forment un espace destiné à recevoir un fluide moteur provoquant une variation du volume de la vessie 23 afin d'induire une déformation de celle-ci. L'alimentation de la vessie 23 en fluide moteur est réalisée grâce à un vérin hydraulique M connecté à la vessie 23 par l'intermédiaire de flexibles d'alimentation hydraulique 233.

La cavité de l'autre 22 des demi-coques - par exemple la demi-coque mobile en rotation - est destinée à recevoir un coussin amortisseur déformable à volume constant 24. Ce coussin 24 est dit « coussin passif» en ce qu'il n'est pas alimenté en fluide moteur. Sa déformation est liée aux forces appliquées sur celui-ci. Le fait que la demi-coque mobile 22 soit agencée pour recevoir un coussin passif 24 permet d'éviter la présence d'une alimentation hydraulique (pour le passage du fluide moteur) sur la demi-coque mobile. Ceci permet de diminuer l'encombrement du dispositif d'injection puisqu'il n'est pas nécessaire de prévoir un dégagement pour que des flexibles d'alimentation hydraulique 233 puissent se déplacer avec la demi-coque mobile 22. Ceci permet également de faciliter la manipulation du dispositif d'injection puisqu'il n'est plus nécessaire pour l'utilisateur d'assister le déplacement des flexibles d'alimentation hydraulique.

Les demi-coques 21, 22 peuvent être réalisées, par exemple, en aluminium ou en matériau composite avec de la fibre de verre ou de la fibre de carbone.

Avantageusement, les demi-coques 21, 22 peuvent s'ouvrir dans deux positions :
- une première position de préparation où les deux demi-coques forment un angle compris entre 10° et 45° l'une par rapport à l'autre ; cette première position d'ouverture permet l'insertion d'une poche dans le dispositif d'injection,
- une deuxième position de maintenance où les deux demi-coques forment un angle compris entre 85 et 95° l'une par rapport à l'autre, et de préférence égal à 90° ; cette deuxième position d'ouverture permet le nettoyage du dispositif d'injection.

### Vessie

L'une des membranes 232 de la vessie 23 - dite « membrane de fond » - est destinée à venir en regard de la face avant de la demi-coque.

De préférence, la forme de la membrane de fond 232 est complémentaire de la forme de la face avant de la demi-coque. Par exemple, dans un mode de réalisation, la membrane de fond 232 et la face avant sont en forme de goutte (cf. figure 8). Ceci permet de limiter la quantité de fluide moteur à introduire dans (respectivement extraire de) la vessie 23 pour augmenter (respectivement diminuer) son volume. On limite ainsi l'encombrement du dispositif d'injection, et on améliore la réactivité du dispositif à vitesse de remplissage donnée.

L'autre membrane 231 - dite membrane frontale - est destinée à venir en regard de la poche 4.

La rigidité de la membrane de fond 232 peut être prévue supérieure à la rigidité de la membrane frontale 231. Par exemple :
- la membrane frontale 231 peut être souple et présenter une dureté shore de l'ordre de 20 à 50 shore A (unité reconnue notamment par les normes ISO 868 et 7619, ASTM D 2240 et DIN 53505),
- la membrane de fond 232 peut être semi-rigide et présenter une dureté shore comprise entre 70 et 90 shore A.

Le fait que la membrane de fond 232 présente une rigidité supérieure à la membrane frontale 231 permet :
- d'une part d'obtenir un bon placage de la membrane de fond 232 contre la paroi de fond 211 de la demi-coque même à faible pression,
- d'autre part de garantir que, lors du retrait de fluide moteur dans la vessie 23, ce soit la paroi frontale 231 de la vessie 23 qui se déforme.

La vessie 23 comprend également une ouverture 234 - par exemple dans la membrane de fond 232 - pour le passage du fluide moteur. L'introduction (respectivement le retrait) de fluide moteur dans (respectivement de) la vessie 23 induit une variation (augmentation ou diminution) de son volume qui provoque une déformation de celle-ci.

Enfin, la vessie 23 comprend un squelette de maintien 236 à sa périphérie pour rigidifier la vessie de sorte à maintenir sa forme - selon le plan P, plan passant par les zones de contact entre les deux demi-coques 21, 22 - notamment lors du retrait de fluide moteur. Ce squelette de maintien est par exemple métallique.

La vessie peut également comprendre un corps de renfort 235 à la périphérie des membranes de la vessie 23. Ce corps de renfort 233 est par exemple en textile. La présence d'un corps de renfort 233 à la périphérie des membranes de la vessie 23 permet d'éviter la formation d'une hernie (i.e. bourrelet) entre les deux demi-coques 21, 22 durant l'introduction de fluide moteur dans la vessie lorsque les demi-coques sont dans la position fermée.

### Coussin

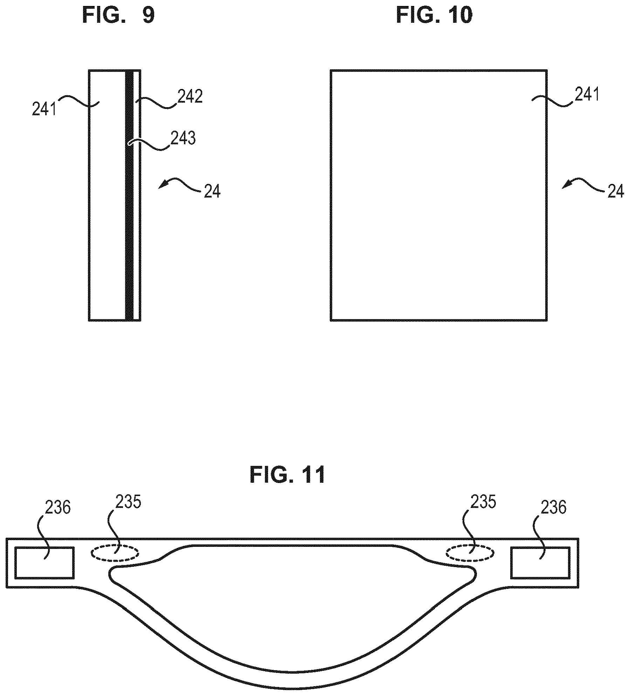

En référence aux figures 9 et 10, le coussin passif 24 comprend une couche épaisse souple 241. Cette couche épaisse 241 est de préférence constituée dans un matériau de dureté shore A nulle tel qu'un gel, et éventuellement de forte conductivité thermique.

Le matériau constituant la couche épaisse 241 est par exemple du silicone ou du polyuréthane.

La couche épaisse 241 peut être recouverte d'une couche mince anti-adhérente pour limiter les frottements entre la poche et le coussin passif 24. Cette couche mince est par exemple une couche de peinture polyuréthane ou une enveloppe de coton ou de Lycra®.

Le coussin passif 24 peut comprendre une face arrière rigide 242 destinée à venir en regard de la face avant de la demi-coque. Dans ce cas, la face arrière rigide 242 présente une forme conjuguée de la forme de la face avant de la demi-coque. La présence d'une face arrière rigide 242 sur le coussin 24 permet de faciliter sa manipulation et sa fixation sur la demi-coque mobile.

Le coussin passif 24 peut également comprendre un (ou plusieurs) élément(s) chauffant 243 composé(s) par exemple d'une couche isolante et d'une couche résistive, ou tout autre type d'élément chauffant connu de l'homme du métier. La présence d'un élément chauffant 243 permet de maintenir le produit liquide contenu dans la poche à une température désirée préalablement à son injection dans le patient.

De préférence, l'élément chauffant 243 est positionné entre la face arrière rigide 242 et la couche épaisse souple 241. En effet :
- l'élément chauffant étant non extensible, et
- la couche épaisse étant destinée à être déformée,
il est préférable de positionner l'élément chauffant entre la face arrière rigide 242 et la couche épaisse souple 241 pour limiter les risques de détérioration de celui-ci.

### Logement d'embout de poche

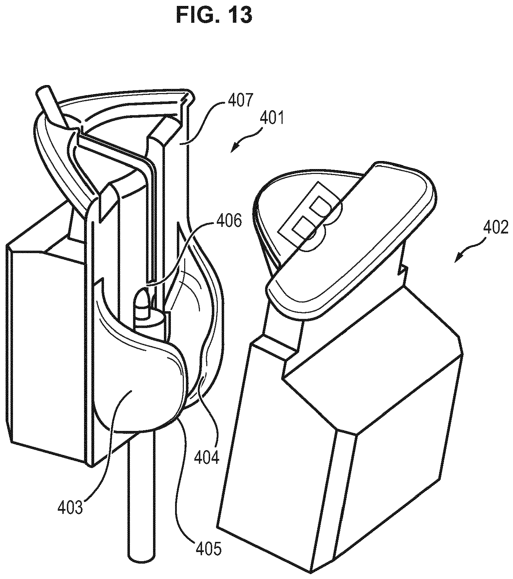

Comme illustré à la figure 13, le dispositif d'injection peut également comprendre un logement d'embout de poche en deux parties disposées chacune sur une demi-coque 21, 22 respective. L'une 401 des parties de ce logement est destinée à recevoir le raccord de branchement du membre d'accès 43 de la poche 4, ou tout type d'élément de couplage positionné à l'extrémité distale du membre d'accès 43. L'autre 402 des parties de ce logement est destinée à assurer le blocage en position du raccord de branchement lorsque le dispositif est dans la position fermée.

La partie du logement destinée à recevoir le raccord de branchement sera dénommée « portion réceptrice » dans la suite de la présente description.

La portion réceptrice 401 comprend une paroi latérale 403 présentant de préférence une symétrie de révolution. Ceci permet d'assurer un bon positionnement de la poche dans l'unité de mise en pression par gravité sans requérir une attention particulière de la part de l'utilisateur lors de la mise en place. La paroi latérale de la portion réceptrice présente par exemple la forme d'un tronc de cône (tel un entonnoir) ou encore d'un cylindre.
La paroi latérale de la portion réceptrice 401 présente en outre une fente longitudinale 404 formant guide pour le passage de l'extrémité d'une tubulure couplée au membre d'accès. Le guide et la forme de la paroi latérale sont adaptées pour permettre le glissement par gravité du raccord de branchement vers le fond 405 de la portion réceptrice. Ceci permet d'assurer un bon positionnement de la tubulure le long du dispositif d'injection.

De préférence, la portion réceptrice 401 comprend également une butée 406 adaptée pour empêcher un mouvement du raccord de branchement lorsque le raccord de branchement est en position dans la portion réceptrice. Plus précisément, la butée permet de prévenir un mouvement du raccord vers le sommet 407 de la portion réceptrice lorsque le raccord de branchement est en position au fond de la portion réceptrice. La butée fait par exemple saillie vers l'intérieur du logement perpendiculaire à l'axe longitudinal de la portion réceptrice.

La partie du logement destinée à assurer le blocage en position du raccord de branchement peut comprendre un doigt faisant saillie vers l'extérieur et destiné à venir se loger dans la fente longitudinale lorsque les deux demi-coques sont dans la position fermée. Ceci permet d'assurer un bon placage de la tubulure dans la fente longitudinale.

Un capteur de bulle peut être prévu au niveau de cette fente longitudinale pour permettre la détection de bulles dans la tubulure reliée au patient. Le capteur de bulle peut être utilisé lors de la phase d'injection de produit liquide au patient pour éviter les risques liés à l'injection d'air au patient. Ce capteur de bulle peut également être utilisé lors des phases de remplissage de la poche contenue dans l'injecteur, notamment pour faciliter les phases de purge, comme il sera décrit plus en détail dans la suite.

### Connectiaue de remplissage

Le dispositif d'injection de type injecteur à poche décrit ci-dessus peut être utilisé avec un ensemble de connexion permettant l'injection et le remplissage de la poche pour produit de contraste. Dans le domaine de l'injection d'un produit liquide - tel que du produit de contraste - à un patient, la phase d'injection est précédée par une phase de remplissage au cours de laquelle le produit liquide est transféré d'un contenant initial - tel qu'un flacon - vers une poche vide.

Il existe des dispositifs permettant d'effectuer un remplissage d'une poche vide à partir d'un contenant initial. Un inconvénient de ces dispositifs est qu'ils sont indépendants de l'injecteur, ce qui nécessite des manipulations de la part de l'utilisateur et diminue l'asepsie.

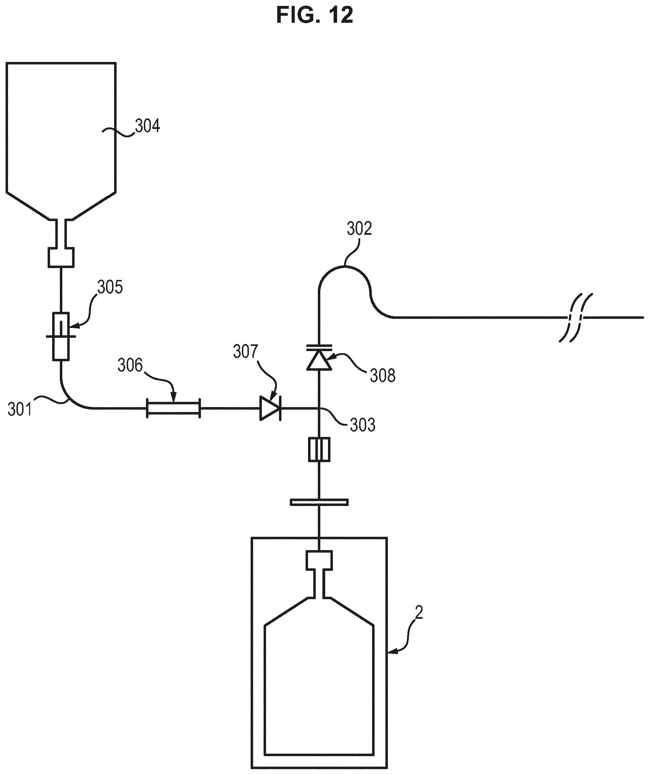

En référence à la figure 12, on a illustré un ensemble de connexion permettant une intégration d'un dispositif de remplissage à un injecteur à poche.

L'ensemble de connexion comprend une ligne de connexion amont 301 et une ligne de connexion aval 302. Les lignes amont et aval sont reliées par l'une de leurs extrémités à une poche contenue dans l'injecteur. Cette liaison entre la poche et les lignes amont et aval est obtenue en utilisant un connecteur 303 à trois entrées de type connecteur en Y. Les autres extrémités des lignes amont et aval sont reliées respectivement :
- à une source de produit liquide 304 pour la ligne amont, et
- à un patient (non représenté) pour la ligne aval.

La fonction de la ligne amont est de permettre le transfert du produit liquide depuis une source initiale vers la poche lors d'une phase de remplissage de la poche.

La ligne amont peut comprendre un piégeur de bulle 305 - tel qu'un compte-goutte - entre la source et la poche. Ce piégeur de bulle permet de chasser l'air entre la source et la poche à remplir.

La ligne amont peut également comprendre une interface de pompage 306 entre le piégeur de bulle et la poche. Cette interface de pompage est par exemple un tuyau souple dont le diamètre, l'épaisseur et l'élasticité permettent un meilleur couplage avec une unité de pompage telle qu'une pompe péristaltique.

La ligne amont peut aussi comprendre un clapet anti-retour 307 entre l'interface de pompage et la poche. Ce clapet anti-retour permet le passage de liquide dans un unique sens, à savoir de la source vers la poche.

La fonction de la ligne aval est de permettre le transfert de liquide entre la poche et le patient lors d'une phase d'injection.

La ligne aval peut comprendre un clapet anti-retour 308 entre la poche et le patient. Ce clapet anti-retour permet le passage de liquide dans un sens unique, à savoir de la poche vers le patient. Avantageusement, le clapet anti-retour peut présenter un seuil d'ouverture élevé (i.e. supérieur à 500 mbar). Ceci permet d'éviter les risques de transfert de produit liquide directement vers le patient (sans passer par la poche) lors d'une phase de remplissage, notamment accélérée. Ceci permet également une mise en pression de la poche souple garantissant l'absence d'air entre la poche et l'injecteur avant le démarrage du transfert de liquide vers la ligne aval.

La ligne aval peut être reliée au patient par l'intermédiaire d'un cathéter.

L'ensemble de connexion décrit ci-dessus permet de réaliser des transferts rapides d'un contenant primaire de fluide vers une poche souple tout en limitant les risques de contamination par l'utilisateur.

Le lecteur aura compris que de nombreuses modifications peuvent être apportées sans sortir matériellement des nouveaux enseignements et des avantages décrits ici.

Par exemple, dans le cas d'une unité de mise en pression de type injecteur à poche, le dispositif d'injection peut comprendre deux paires de demi-coquilles permettant l'injection successive ou simultanée de différents produits liquides injectables.

Par ailleurs, l'ensemble de connexion illustré à la figure 12 était présenté en référence à l'injecteur à poche selon l'invention. L'homme du métier appréciera que cet ensemble de connexion peut être utilisé avec un autre type d'injecteur à poche de l'art antérieur, ou d'autres types d'injecteur tel qu'un injecteur dit « pousse seringue ».

Par conséquent, toutes les modifications de ce type sont destinées à être incorporées à l'intérieur de la portée des revendications jointes.

## Revendications

1. Dispositif pour l'injection d'un produit liquide contenu dans une poche, le dispositif comprenant un boîtier constitué de deux demi-coques (21, 22) articulées afin de permettre le déplacement relatif des demi-coques l'une par rapport à l'autre entre :
- une position ouverte pour la mise en place de la poche et
- une position fermée pour l'injection du produit liquide contenu dans la poche,
**caractérisé en ce que** :
- l'une des demi-coques comprend une vessie (23) - dite « vessie active » - déformable à volume variable sous l'action d'une source de puissance hydraulique alimentant ladite vessie en fluide hydraulique, et
- l'autre des demi-coques comprend un coussin (24) déformable à volume constant - dit « coussin passif ».

2. Dispositif pour injection selon la revendication 1, dans lequel chaque demi-coque comporte une cavité de sorte à former un berceau pour la vessie active ou le coussin passif.

3. Dispositif selon l'une quelconque des revendications précédentes, dans lequel la demi-coque comprenant la vessie active est fixe et la demi-coque comprenant le coussin passif est mobile en rotation.

4. Dispositif selon l'une quelconque des revendications précédentes, dans lequel la vessie active comprend deux membranes superposées fixées à leurs périphéries de manière à obtenir un volume étanche destiné à recevoir le fluide hydraulique.

5. Dispositif selon la revendication 4, dans lequel la membrane (232) destinée à venir en contact avec la demi-coque présente une rigidité supérieure à l'autre membrane (231) destinée à venir en contact avec la poche.

6. Dispositif selon la revendication 5, dans lequel la dureté shore de la membrane (232) destinée à venir en contact avec la demi-coque est comprise entre 70 et 90 shore A, la dureté shore de l'autre membrane (231) étant comprise entre 20 et 50 shore A.

7. Dispositif selon l'une des revendications 4 à 6, dans lequel la vessie comprend en outre un corps annulaire (233) de renfort rigide s'étendant à la périphérie des membranes de la vessie.

8. Dispositif selon l'une quelconque des revendications précédentes, dans lequel le coussin passif comprend une couche épaisse déformable (241) de dureté shore inférieure à 10 shore A.

9. Dispositif selon la revendication 8, dans lequel le coussin passif comprend en outre une couche rigide (242) superposée à la couche épaisse, la couche rigide étant destinée à venir en contact avec la demi-coque.

10. Dispositif selon la revendication 9, dans lequel le coussin passif comprend un élément chauffant (243) positionné entre la couche rigide et la couche épaisse déformable.

11. Dispositif selon l'une quelconque des revendications 8 à 10, dans lequel le coussin passif comprend en outre une structure de maintien rigide s'étendant à la périphérie de la couche épaisse déformable.

12. Dispositif selon l'une quelconque des revendications précédentes, dans lequel l'une des demi-coques comprend un logement comportant une paroi latérale, le logement étant destiné à recevoir un raccord de branchement d'un membre d'accès de la poche, la forme de la paroi latérale dudit logement présentant une symétrie de révolution.

13. Dispositif selon la revendication 12, dans lequel le logement comprend un guide, tel qu'une fente longitudinale, pour le passage du membre d'accès de la poche, le guide et la forme de la paroi latérale étant adaptés pour permettre le positionnement du raccord de branchement au niveau d'une base du logement par gravité.

14. Dispositif selon l'une quelconque des revendications 12 ou 13, dans lequel le logement comprend en outre une butée adaptée pour empêcher un mouvement du raccord de branchement selon une direction parallèle à un axe longitudinal du logement lorsque le raccord de branchement est en position dans le logement.

15. Dispositif selon l'une quelconque des revendications précédentes, lequel comprend en outre une unité de contrôle programmée pour :
- recevoir une information sur la nature du produit liquide contenu dans la poche,
- déterminer un débit de sortie de référence en fonction de ladite information sur la nature du produit contenu dans la poche,
- estimer le débit de sortie du dispositif d'injection,
- comparer le débit de sortie de référence au débit de sortie estimé,
- émettre une alarme si la différence entre le débit de sortie estimé et le débit de sortie de référence est supérieure à une valeur seuil.

## Patentansprüche

1. Vorrichtung zur Injektion eines Flüssigprodukts, das in einem Beutel enthalten ist, wobei die Vorrichtung ein Gehäuse umfasst, das aus zwei Halbschalen (21, 22) besteht, die angelenkt sind, um das relative Verlagern der Halbschalen zueinander zu erlauben zwischen:
- einer offenen Position zum Anbringen des Beutels und
- einer geschlossenen Position zum Injizieren des Flüssigprodukts, das in dem Beutel enthalten ist, **dadurch gekennzeichnet, dass**
- eine der Halbschalen eine Blase (23), "aktive Blase" genannt, umfasst, die mit variablem Volumen unter der Einwirkung einer hydraulischen Leistungsquelle, die die Blase mit Hydraulikflüssigkeit versorgt, verformbar ist, und
- die andere der Halbschalen ein Kissen (24) umfasst, das auf ein konstantes Volumen verformbar ist, "passives Kissen" genannt.

2. Vorrichtung zur Injektion nach Anspruch 1, wobei jede Halbschale einen Hohlraum derart umfasst, dass eine Wiege für die aktive Blase oder das passive Kissen gebildet wird.

3. Vorrichtung nach einem der vorhergehenden Ansprüche, wobei die Halbschale, die die aktive Blase umfasst, stationär ist, und die Halbschale, die das passive Kissen umfasst, in Drehung beweglich ist.

4. Vorrichtung nach einem der vorhergehenden Ansprüche, wobei die aktive Blase zwei übereinanderliegende Membranen umfasst, die an ihren Umfängen derart befestigt sind, dass ein dichtes Volumen erhalten wird, das dazu bestimmt ist, die Hydraulikflüssigkeit aufzunehmen.

5. Vorrichtung nach Anspruch 4, wobei die Membran (232), die dazu bestimmt ist, mit der Halbschale in Berührung zu kommen, eine Steifigkeit aufweist, die größer ist als die andere Membran (231), die dazu bestimmt ist, mit dem Beutel in Berührung zu kommen.

6. Vorrichtung nach Anspruch 5, wobei die Shore-Härte der Membran (232), die dazu bestimmt ist, mit der Halbschale in Berührung zu kommen, zwischen 70 und 90 Shore A liegt, wobei die Shore-Härte der anderen Membran (231) zwischen 20 und 50 Shore A liegt.

7. Vorrichtung nach einem der Ansprüche 4 bis 6, wobei die Blase außerdem einen starren ringförmigen Verstärkungskörper (233) umfasst, der sich an der Peripherie der Membranen der Blase erstreckt.

8. Vorrichtung nach einem der vorhergehenden Ansprüche, wobei das passive Kissen eine dicke verformbare Schicht (241) mit Shore-Härte kleiner als 10 Shore A umfasst.

9. Vorrichtung nach Anspruch 8, wobei das passive Kissen außerdem eine starre Schicht (242) umfasst, die der dicken Schicht überlagert ist, wobei die starre Schicht dazu bestimmt ist, mit der Halbschale in Berührung zu kommen.

10. Vorrichtung nach Anspruch 9, wobei das passive Kissen ein Heizelement (243) umfasst, das zwischen der starren Schicht und der verformbaren dicken Schicht positioniert ist.

11. Vorrichtung nach einem der Ansprüche 8 bis 10, wobei das passive Kissen außerdem eine starre Haltestruktur umfasst, die sich an dem Umfang der verformbaren dicken Schicht erstreckt.

12. Vorrichtung nach einem der vorhergehenden Ansprüche, wobei eine der Halbschalen eine Aufnahme umfasst, die eine Seitenwand umfasst, wobei die Aufnahme dazu bestimmt ist, einen Anschluss eines Zugangselements des Beutels aufzunehmen, wobei die Form der Seitenwand der Aufnahme eine Rotationssymmetrie aufweist.

13. Vorrichtung nach Anspruch 12, wobei die Aufnahme eine Führung, wie einen Längsschlitz, für das Durchgehen des Zugangselements des Beutels umfasst, wobei die Führung und die Form der Seitenwand angepasst sind, um das Positionieren des Anschlusses im Bereich einer Basis der Aufnahme schwerkraftbedingt zu erlauben.

14. Vorrichtung nach einem der Ansprüche 12 oder 13, wobei die Aufnahme außerdem einen Anschlag umfasst, der angepasst ist, um eine Bewegung des Anschlusses entlang einer Richtung parallel zu einer Längsachse der Aufnahme zu verhindern, wenn der Anschluss in der Aufnahme in Position ist.

15. Vorrichtung nach einem der vorhergehenden Ansprüche, die außerdem eine Steuereinheit umfasst, die programmiert ist, um:
- eine Information über die Beschaffenheit des Flüssigprodukts, das in dem Beutel enthalten ist, zu empfangen,
- einen Referenzausgangsdurchsatz in Abhängigkeit von der Information über die Beschaffenheit des Produkts, das in dem Beutel enthalten ist, zu bestimmen,
- den Ausgangsdurchsatz der Injektionsvorrichtung zu schätzen,
- den Referenzausgangsdurchsatz mit dem geschätzten Ausgangsdurchsatz zu vergleichen,
- einen Alarm abzugeben, falls der Unterschied zwischen dem geschätzten Ausgangsdurchsatz und dem Referenzausgangsdurchsatz größer ist als ein Schwellenwert.

## Claims

1. Device for the injection of a liquid product contained in a sachet, the device comprising a casing constituted of two half-shells (21, 22) articulated in order to enable relative movement of the half-shells one relative to the other between:
- an open position for putting the sachet in place, and
- a closed position for injection of the liquid product contained in the sachet,
**characterized in that**:
- one of the half-shells comprises a variable volume bladder (23) - called the "active bladder" - deformable by a source of hydraulic power feeding said bladder with hydraulic fluid, and
- the other half-shell comprises a constant volume deformable cushion (24) - called the "passive cushion".

2. Injection device according to Claim 1, wherein each half-shell includes a cavity so as to form a cradle for the active bladder or the passive cushion.

3. Device according to either one of the preceding claims, wherein the half-shell comprising the active bladder is fixed and the half-shell comprising the passive cushion is mobile in rotation.

4. Device according to any one of the preceding claims, wherein the active bladder comprises two superposed membranes fixed at their peripheries so as to obtain a fluid-tight volume designed to receive the hydraulic fluid.

5. Device according to Claim 4, wherein the membrane (232) designed to come into contact with the half-shell has a higher stiffness than the other membrane (231) designed to come into contact with the sachet.

6. Device according to Claim 5, wherein the Shore A hardness of the membrane (232) designed to come into contact with the half-shell is from 70 to 90 and the Shore A hardness of the other membrane (231) is from 20 to 50.

7. Device according to any one of Claims 4 to 6, wherein the bladder further comprises a rigid annular reinforcing body (233) at the periphery of the membranes of the bladder.

8. Device according to any one of the preceding claims, wherein the passive cushion comprises a thick deformable layer (241) with a Shore A hardness less than 10.

9. Device according to Claim 8, wherein the passive cushion further comprises a rigid layer (242) superposed on the thick layer, the rigid layer being designed to come into contact with the half-shell.

10. Device according to Claim 9, wherein the passive cushion comprises a heating element (243) disposed between the rigid layer and the thick deformable layer.

11. Device according to any one of Claims 8 to 10, wherein the passive cushion further comprises a rigid holding structure extending to the periphery of the deformable thick layer.

12. Device according to any one of the preceding claims, wherein one of the half-shells comprises a housing including a lateral wall, the housing being designed to receive a connector for connecting an access member of the sachet, the shape of the lateral wall of said housing having symmetry of revolution.

13. Device according to Claim 12, wherein the housing comprises a guide, such as a longitudinal slot, for the access member of the sachet to pass through, the guide and the shape of the lateral wall being adapted to enable positioning of the connector at the level of a base of the housing by gravity.

14. Device according to either one of Claims 12 or 13, wherein the housing further comprises a stop adapted to prevent movement of the connector in a direction parallel to a longitudinal axis of the housing when the connector is in position in the housing.

15. Device according to any one of the preceding claims, which further comprises a control unit programmed:
- to receive information on the nature of the liquid product contained in the sachet,
- to determine a reference rate of outflow as a function of said information on the nature of the product contained in the sachet,
- to estimate the rate of outflow from the injection device,
- to compare the reference rate of outflow to the estimated rate of outflow,
- to emit an alarm if the difference between the estimated rate of outflow and the reference rate of outflow is greater than a threshold value.
